# EUROPÄISCHE PATENTSCHRIFT

(11) **EP 3 145 501 B1**
(45) Veröffentlichungstag und Bekanntmachung des Hinweises auf die Patenterteilung: **21.02.2024**
(21) Anmeldenummer: 15720604.6
(22) Anmeldetag: 08.05.2015
(51) Int. Cl.: A61K 9/70, A61K 36/53, A61P 25/20, A61P 25/00, A61P 25/22

(54) **LAVENDELÖL ENTHALTENDES TRANSDERMALES THERAPEUTISCHES SYSTEM**
TRANSDERMAL THERAPEUTIC SYSTEM CONTAINING LAVENDER OIL
SYSTÈME THÉRAPEUTIQUE TRANSDERMIQUE CONTENANT DE L'HUILE DE LAVANDE

(30) Priorität: 20.05.2014 EP 14169037
(43) Veröffentlichungstag der Anmeldung: 29.03.2017
(73) Patentinhaber: LTS Lohmann Therapie-Systeme AG, 56626 Andernach (DE)
(72) Erfinder: BOTZEM, Petra, 56626 Andernach (DE); HILLE, Thomas, 56567 Neuwied (DE)
(74) Vertreter: Schweitzer, Klaus
(86) Internationale Anmeldenummer: PCT/EP2015/000955
(87) Internationale Veröffentlichungsnummer: WO 2015/176801

(56) Entgegenhaltungen:
- WO-A1-00/56290
- WO-A1-98/17262
- CN-A- 103 316 078
- DE-A1- 10 246 377
- DE-A1-102005 010 255
- US-A1- 2011 300 198
- PHARMAZEUTISCHEN ZEITUNG, vol. 37, no. 99, 1 January 1892 (1892-01-01), pages 764-765,

## Beschreibung

Die Erfindung betrifft ein den Wirkstoff Lavendelöl enthaltendes transdermales therapeutisches System (TTS) und seine Verwendung bei der Prophylaxe und/oder Behandlung von Unruhezuständen, Schlafstörungen, Ängsten und Nervosität, insbesondere von Unruhezuständen bei ängstlicher Verstimmung.

Die Erfindung betrifft auch Verfahren zur Herstellung dieser transdermalen therapeutischer Systeme, bei denen die Systeme unter Verwendung von Stützmaterialien aus faserförmigen Bestandteile hergestellt und mittels eines Druckverfahrens mit Wirkstoff beladen werden.

Aus der US 2008/124410 A1 sind die prophylaktische und therapeutische Verwendung von Lavendelöl zur Behandlung von Neurasthenie, Somatisierungsstörungen und von anderen stress-assoziierten Erkrankungen sowie Lavendelöl enthaltende Arzneimittel und diätetische Lebensmittel als auch Zubereitungen und Kapseln als orale Darreichungsformen bekannt.

WO 00/56290 A1 offenbart ein transdermales therapeutisches System (TTS), das als wesentliche Merkmale eine der Haut abgewandte, für den Wirkstoff undurchlässige Rückschicht, mindestens ein Wirkstoffdepot, eine Matrix, die mit dem Wirkstoffdepot in Verbindung steht und die Abgabe des Wirkstoffs steuert, und eine haftklebende Fixierungseinrichtung für das therapeutische System auf der Haut enthält, wobei das Depot und/oder die Matrix noch Stützmaterialien aus Papier enthalten.

Aus der DE 102 46 377 A1 ist ein Verfahren zur Herstellung eines wirkstoffhaltigen Pflasters bekannt. In diesem Verfahren wird ein saugfähiges Trägermaterial auf einer Seite mit einer Selbstklebemasse beschichtet und auf der gegenüberliegenden Seite mit einem Wirkstoff oder einer wirkstoffhaltigen Lösung beaufschlagt, so dass das Trägermaterial eine Menge des Wirkstoffes oder der wirkstoffhaltigen Lösung aufnimmt. Die mit Klebmasse beschichtete Seite des Trägermaterials wird über seine ganze Breite mit einem klebstoffabweisenden Trägermaterial abgedeckt und das gesamte Pflaster luftdicht eingesiegelt.

WO 98/17262 A1 offenbart eine Vorrichtung, die zur topischen Abgabe von Aromaöl an den Körper geeignet ist. Die Vorrichtung umfasst einen Verband, der ein Aromaöl enthält. Der Verband liegt vorzugsweise in Form eines selbstklebenden Hautpflasters vor, der die Übertragung des Öls über längere Zeiträume erleichtert und den Verlust von Ölen durch Verdunstung vermeidet. Das Aromaöl umfasst idealerweise ein oder mehrere ätherische Öle, die mit einem Trägeröl gemischt sind. Das Trägeröl unterstützt nicht nur das Eindringen des ätherischen Öls durch die Haut, sondern verstärkt auch synergistisch die therapeutische Wirkung der ätherischen Öle.

US 2011/300198 A1 offenbart dermale Pflaster, die natürliche Polysaccharide umfassen und keine synthetischen Haftkleber enthalten. Die dermalen Pflaster umfassen weiterhin mindestens ein ätherisches Öl, ein Co-Lösungsmittel, einen Emulgator und Wasser.

CN 103 316 078 A offenbart Pflaster, die aus einer Rückschicht, einer Gelschicht und einer ablösbaren Schutzschicht aufgebaut sind und die bei der Behandlung von Schlafstörungen und als kühlendes Pflaster Verwendung finden. Die Pflaster enthalten ein Wirkstoffgemisch aus den drei Wirkstoffen Asbolin, Lavendelöl und Menthol, die zu je 0,01 bis 0,05 Teilen vorliegen.

Bei der oralen Verabreichung von Lavendelöl kann allerdings gelegentlich Übelkeit sowie ein Aufstoßen und ein Atemgeruch nach Lavendelöl auftreten.

Aufgrund ihrer möglicherweise kurzen Halbwertzeit und ihres First-pass-Effekts kann die orale Verabreichung von Wirkstoffen problematisch sein. Eine kurze Halbwertzeit würde dabei eine oftmalige Einnahme der Substanz und ein hoher First-pass-Effekt eine hohe Dosierung nötig machen. Während die Einnahmefrequenz durch eine geeignete orale Formulierung möglicherweise überwunden werden kann, ist das Problem eines hohen First-pass-Effekts prinzipiell nur durch eine nicht-orale Zufuhr des Wirkstoffs zu lösen.

Aufgabe der vorliegenden Erfindung ist, ein Mittel bereitzustellen, das wirksam zur Prophylaxe und/oder Behandlung von Unruhezuständen, Schlafstörungen, Ängsten und Nervosität verwendet werden kann, das die genannten Nachteile nicht aufweist und das weitgehend frei von Nebenwirkungen ist.

Diese Aufgabe wird durch ein den Wirkstoff Lavendelöl enthaltendes transdermales therapeutisches System (TTS) gelöst.

Transdermale therapeutische Systeme sind Systeme zur kontrollierten Verabreichung von pharmazeutischen Wirkstoffen über die Haut. Sie werden seit längerem zur Behandlung unterschiedlicher Krankheiten, körperlicher wie geistiger Funktionsstörungen, Beschwerden sowie Unpässlichkeiten eingesetzt. Bei transdermalen therapeutischen Systemen handelt es sich um schichtförmig aufgebaute Erzeugnisse in Form von Pflastern, die eine wirkstoffundurchlässige Rückschicht, mindestens eine wirkstoffhaltige Reservoir- oder Matrixschicht, gegebenenfalls eine die Geschwindigkeit der Wirkstofffreisetzung kontrollierende Membran und eine ablösbare Schutzschicht, die vor Gebrauch des TTS von diesem abgezogen wird, umfassen.

Zur Befestigung eines transdermalen therapeutischen Systems auf der Haut sowie zur Gewährleistung der kontrollierten Verabreichung des Wirkstoffs ist das TTS mit einer haftklebenden Schicht versehen. Diese haftklebende Schicht kann mit der wirkstoffhaltigen Matrixschicht oder der hautseitigen wirkstoffhaltigen Schicht identisch sein, kann aber auch zusätzlich vorhanden sein, sofern die (hautseitige) wirkstoffhaltige Schicht oder die optional vorhandene Membran nicht haftklebend ist.

Die Rückschicht eines TTS muss für den im TTS enthaltenen Wirkstoff undurchlässig sein, um ein unerwünschtes Austreten des Wirkstoffs aus der Seite des TTS, die der Haut abgewandt ist, zu verhindern. Hierfür werden insbesondere Metallfolien, spezielle Kunststofffolien sowie Verbundlaminate dieser Werkstoffe eingesetzt. Am gebräuchlichsten sind Verbundlaminate aus Aluminium und Kunststoffmaterialien wie Polyethylenterephthalat. Der Vorteil dieser Verbundlaminate liegt darin, dass Aluminiumfolien kostengünstig herzustellen und gegenüber nahezu allen pharmazeutischen Wirkstoffen undurchlässig sind. Zudem sind Aluminiumfolien lichtundurchlässig, was gerade bei lichtempfindlichen Wirkstoffen den Vorteil eines zuverlässigen Schutzes vor Licht bietet.

Ein TTS zur Abgabe von wenigstens einem pharmazeutischen Wirkstoff, das wenigstens einen eingeschlossenen, vorzugsweise verkapselten Duftstoff, wie unter anderem Lavendelöl, aufweist, der spätestens beim Aufbringen des Systems freigesetzt wird, wird in der WO 2007/006529 A1 beschrieben. Der Duftstoff dient dazu, die Akzeptanz zur Anwendung des TTS beim Patienten zu verbessern, aber nicht zur systemischen Gabe von Lavendelöl.

Die Erfindung betrifft transdermales therapeutische Systeme (TTS), umfassend eine der Haut abgewandte, für den Wirkstoff undurchlässige Rückschicht,
a) ein Wirkstoffdepot, das einen Wirkstoff enthält, wobei der Wirkstoff Lavendelöl ist oder die reinen Wirkstoffe Linalool oder Linalylacetat,
b) eine Matrix, die mit dem Wirkstoffdepot in Verbindung steht und die Abgabe des Wirkstoffs steuert,
c) eine haftklebende Fixierungseinrichtung für das therapeutische System auf der Haut und
d) eine ablösbare Schutzschicht,
   worin die Matrix und die haftklebende Fixiereinrichtung gleich oder verschieden sind und ein Material umfassen, das aus der Gruppe ausgewählt ist, die aus haftklebenden Polymeren auf Basis von Acrylsäure und/oder Metharylsäure sowie deren Estern, Polyacrylaten, Polyisobutylen, Polyvinylacetat, Ethylen-Vinylacetat-copolymer, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymeren und Heißschmelzklebern besteht, oder das auf Basis von haftklebenden Silikonpolymeren oder Polysiloxanen hergestellt ist,
   wobei der Wirkstoffgehalt 30 bis 400 mg beträgt und
   wobei das Wirkstoffdepot noch faserförmige Bestandteile umfassendes Stützmaterial enthält.

Der Echte Lavendel oder Schmalblättrige Lavendel (Lavandula angustifolia, Syn. Lavandula officinalis, Lavandula vera) ist eine Pflanzenart aus der Familie der Lippenblütler (Lamiaceae). Die Pflanze findet hauptsächlich Verwendung als Zierpflanze oder zur Gewinnung von Duftstoffen. Das Verbreitungsgebiet reicht von den Kanaren durch das ganze Mittelmeergebiet bis Vorderindien. Als Inhaltsstoffe sind v. a. ätherische Öle (Lavendelöl) in den oberirdischen Teilen, sowie Kaffeesäure und deren Depside in den Blättern beschrieben. Das Lavendelöl setzt sich zusammen aus Linalylacetat, Linalool, Campher und Cineol. Seine Inhaltsstoffe sind 40 - 50 % Ester, 25 - 35 % Monoterpenole, Monoterpene, Sesquiterpene, Ketone und Oxide. Die aus Lavendel hergestellten ätherischen Öle werden traditionell für kosmetische Produkte, aber auch zu therapeutischen Zwecken z. B. in der Aromatherapie verwendet. So sind für Lavendelöl antibakterielle, antifungale, spasmolytische, sedative und antidepressive Wirkungen beschrieben (H. M. A. Cavanagh et al. (2002), Phytother. Res. 16, 301 - 308).

Lavendelöl kann nach bekannten Herstellungsverfahren, vorzugsweise durch Wasserdampfdestillation von frisch geernteten Lavendelblüten hergestellt werden.

In Deutschland sind Zubereitungen aus Lavendelblüten in Form von Teeaufgüssen, als Extrakt sowie als Badezusatz für die Indikationen Unruhezustände, Einschlafstörungen, funktionelle Oberbauchbeschwerden, Meteorismus und in der Balneotherapie positiv monographiert (Monographie der Kommission E des ehemaligen deutschen Bundesgesundheitsamts).

Das Wirkstoffdepot der erfindungsgemäßen TTS kann Lavendelöl oder die reinen Wirkstoffe Linalool oder Linalylacetat auch in Kombination mit mindestens einen pharmazeutisch akzeptablen Hilfsstoff enthalten. Der Wirkstoffgehalt eines TTS ist 30 bis 400 mg, vorzugsweise 40 bis 300 mg, insbesondere 50 bis 150 mg.

Das Stützmaterial der erfindungsgemäßen TTS umfasst faserförmige Bestandteile, bei welchen es sich um verhältnismäßig lange, dünne und flexible Gebilde aus natürlichem oder synthetischem Material handelt. Zu den faserförmigen Bestandteilen aus natürlichen Materialien zählen beispielsweise Pflanzenfasern, tierische Fasern sowie Mineralfasern. Pflanzenfasern wie Bast, Baumwolle, Hanf, Kokos, Leinen, Kapok oder Ramie bestehen im Allgemeinen aus Zellulose. Zu den tierischen Fasern gehören Seide und Haare (Wolle). Eine natürlich vorkommende Mineralfaser ist Asbest. Zu den synthetischen Fasern zählen Fasern aus Perlon, Nylon, aber auch Kunstseide, Glasfasern und Kohlenstofffasern. Mehrere Fasern bilden gemeinsam größere Strukturen. So können Textilfasern gemeinsam einen Faden, ein Seil oder einen Stoff bilden. Zellulosefasern werden z. B. zur Papier- und Textilherstellung verwendet. Ein bevorzugtes Stützmaterial ist Papier, textiles Material oder ein Vliesstoff.

In einer bevorzugten Ausführungsform weist das Wirkstoffdepot eine Fläche von 10 bis 35 cm², vorzugsweise 12 bis 30 cm², insbesondere 15 bis 25 cm² auf.

Die Matrixschicht und die haftklebende Fixiereinrichtung des erfindungsgemäßen TTS bestehen aus dem gleichen Material oder aus verschiedenen Materialien. Sie umfassen ein Material, das aus der Gruppe ausgewählt ist, die aus haftklebenden Polymeren auf Basis von Acrylsäure und/oder Methacrylsäure sowie deren Estern, Polyacrylaten, Polyisobutylen, Polyvinylacetat, EthylenVinylacetatcopolymer, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymeren und Heißschmelzklebern besteht, oder das auf Basis von haftklebenden Silikonpolymeren oder Polysiloxanen hergestellt ist.

Vorzugsweise ist dieses Material aus der Gruppe ausgewählt, die kationische Copolymere auf Basis von Dimethylaminoethylmethacrylat und neutralen Methacrylsäureestern, beispielsweise Eudragit^{®} E 100, und neutrale Copolymere auf Basis von Butylmethacrylat und Methylmethacrylaten, zum Beispiel Plastoid^{®} B, umfasst.

Als Materialien für die wirkstoffundurchlässige Rückschicht eignen sich vor allem Polyester, die sich durch besondere Festigkeit auszeichnen, wie z. B. Polyethylenterephthalat und Polybutylenterephthalat, darüber hinaus aber nahezu beliebige andere hautverträgliche Kunststoffe, wie Polyvinylchlorid, Polyurethan, Polyvinylidenchlorid, Ethylen-Vinylacetat-Copolymerisate, Polyvinylacetat, Vinylacetat-Vinylchlorid-Copolymerisate, Nylon, Polyethylen, Polypropylen, Polyurethane, Polyamid, Cellulosederivate und viele andere mehr. Bevorzugt sind Polyethylenterephthalat, weichgemachte Vinylacetat-Vinylchlorid-Copolymerisate, Nylon, Ethylen-Vinylacetat-Copolymerisate, weichgemachtes Polyvinylchlorid, Polyurethan, Polyvinylidenchlorid, Polypropylen, Polyethylen und Polyamid. Im Einzelfall kann die Rückschicht mit einer zusätzlichen Auflage versehen werden, z. B. durch Bedampfung mit Metallen, insbesondere Aluminium.

Für die ablösbare Schutzschicht können grundsätzlich dieselben Materialien verwendet werden wie für die Rückschicht, vorausgesetzt, dass sie durch eine geeignete Oberflächenbehandlung, wie z. B. Silikonisierung, ablösbar ausgerüstet sind. Es können aber auch andere ablösbaren Schutzschichten wie z. B. mit Polytetrafluorethylen behandeltes Papier oder ^{®}Cellophan (Cellulosehydrat) verwendet werden.

Das Verfahren zur Herstellung der erfindungsgemäßen TTS ist dadurch gekennzeichnet, dass man
- ein Laminat aus einer wirkstoffundurchlässigen Trägerschicht, einer haftklebenden Fixierschicht und einer Matrixschicht herstellt,
- auf die Matrixseite dieses Laminats eine zur Aufnahme des Wirkstoffs vorgesehene Depotschicht aufbringt,
- mittels eines Druckverfahrens einzeln dosierte Portionen einer fliessfähigen, wirkstoffhaltigen Zubereitung auf diese Depotschicht aufbringt,
- darauf ggf. eine weitere Matrixschicht laminiert und
- das so erhaltene Laminat schließlich mit einer wirkstoffundurchlässigen Rückschicht versieht,
wobei die transdermalen therapeutischen Systeme durch Schneiden und/oder Stanzen vor oder nach dem Aufbringen der wirkstoffhaltigen Zubereitung aus dem bis dahin entstandenen Verbundlaminat vereinzelt werden können.

Bei dem vorstehenden Druckverfahren kann es sich um ein Tampondruckverfahren handeln. Ein solches Verfahren ist aus US-Patent 5 110 599, auf welches vollinhaltlich Bezug genommen wird, bekannt.

Bei dem vorstehenden Druckverfahren kann es sich außerdem um ein Verfahren handeln, bei dem die wirkstoffhaltige Zubereitung durch eine mit mindestens einem Durchlass versehene Verteilerplatte einer Auftragsvorrichtung auf die zur Aufnahme des Wirkstoffs vorgesehene Depotschicht übertragen wird. Ein solches Verfahren ist aus US-Patent 6 187 322, auf welches vollinhaltlich Bezug genommen wird, bekannt.

Der Wirkstoff Lavendelöl kann mittels der beiden vorstehenden Druckverfahren direkt aufgetragen werden. Üblicherweise wird der Wirkstoff jedoch in Form einer Flüssigkeit verwendet, die durch Zugabe geeigneter Lösungsmittel und/oder Hilfsstoffe die gewünschte Viskosität aufweist. Als Lösungsmittel sind prinzipiell alle gängigen organischen Lösungsmittel geeignet, wie zum Beispiel Ethanol, Isopropylalkohol, Heptan, Hexan, Ethylacetat, Petrolether, Benzin, Aceton, Glycerol, DEET (N,N-Diethyl-3-methylbenzamid), THF sowie viele Öle, beispielsweise Silikonöl, Paraffin, Triglyceride, Neutralöl oder pflanzliche Öle. Hilfsstoffe, die die Viskosität des Lavendelöls erhöhen, sind Polymere, die auch zur Herstellung der Matrixschichten verwendet werden. Besonders geeignet ist PVP oder Polymethacrylat. Die Viskosität der als Druckmedium zu verwendenden wirkstoffhaltigen Zubereitung liegt vorzugsweise im Bereich von 10 bis 100 dPa·s, besonders bevorzugt in einem Bereich von 15 bis 25 dPa s.

Die Erfindung betrifft weiterhin Lavendelöl oder die reinen Wirkstoffe Linalool oder Linalylacetat zur Verwendung bei der Prophylaxe und/oder Behandlung von Unruhezuständen, Schlafstörungen, Ängsten und Nervosität, wobei ein TTS umfassend
a) eine der Haut abgewandte, für den Wirkstoff undurchlässige Rückschicht,
b) ein Wirkstoffdepot, das einen Wirkstoff enthält, wobei der Wirkstoff Lavendelöl ist oder die reinen Wirkstoffe Linalool oder Linalylacetat sind,
c) eine Matrix, die mit dem Wirkstoffdepot in Verbindung steht und die Abgabe des Wirkstoffs steuert und
d) eine haftklebende Fixierungseinrichtung für das therapeutische System auf der Haut,
   worin die Matrix und die haftklebende Fixiereinrichtung gleich oder verschieden sind und ein Material umfassen, das aus der Gruppe ausgewählt ist, die aus haftklebenden Polymeren auf Basis von Acrylsäure und/oder Metharylsäure sowie deren Estern, Polyacrylaten, Polyisobutylen, Polyvinylacetat, Ethylen-Vinylacetat-copolymer, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymeren und Heißschmelzklebern besteht, oder das auf Basis von haftklebenden Silikonpolymeren oder Polysiloxanen hergestellt ist,
   wobei der Wirkstoffgehalt 30 bis 400 mg beträgt und worin das Wirkstoffdepot noch faserförmige Bestandteile umfassendes Stützmaterial enthält,
   auf die Haut aufgeklebt wird und der Wirkstoff in prophylaktisch bzw. therapeutisch wirksamer Menge transdermal abgegeben wird, vorzugsweise über einen Zeitraum von mindestens 2 und bis zu 48 Stunden.

Nachfolgend soll die Erfindung anhand eines Ausführungsbeispiels sowie der begleitenden Zeichnungen, in der schematisch der Aufbau erfindungsgemäßer TTS dargestellt ist, erläutert werden, ohne dass die Erfindung darauf beschränkt wäre. Dabei zeigt:
Fig. 1 einen Schnitt durch eine bevorzugte Ausführungsform eines erfindungsgemäßen TTS; und
Fig. 2 einen Schnitt durch eine weitere bevorzugte Ausführungsform eines therapeutischen Systems, bei dem das Wirkstoffdepot sich zwischen Rückschicht und Reservoirmatrix befindet. Dabei ist das TTS nach Abziehen der ablösbaren Schutzschicht auf die Haut aufgeklebt dargestellt.

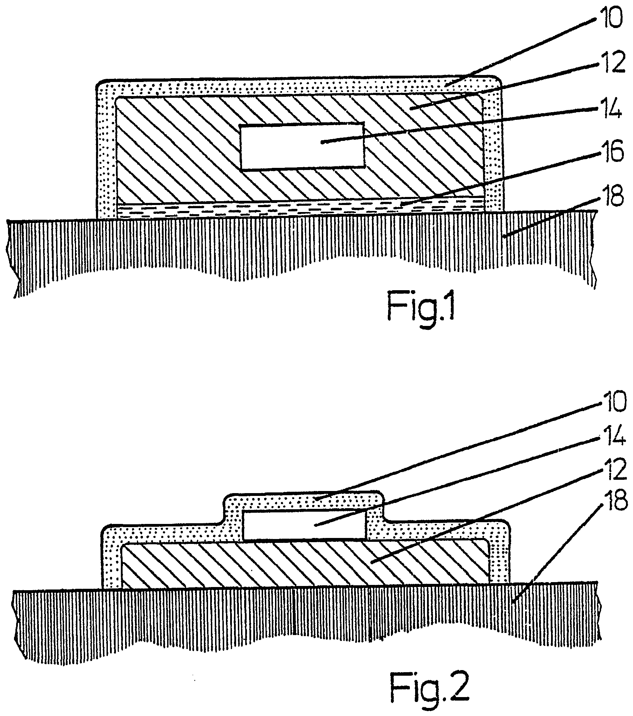

In Fig. 1 ist ein Schnitt durch ein erfindungsgemäßes therapeutisches System, das auf der Haut 18 durch eine Fixierungseinrichtung 16 befestigt ist, schematisch dargestellt. Auf der Fixierungseinrichtung 16 befindet sich die Reservoirmatrixschicht 12, welche bevorzugt zum Zeitpunkt der Herstellung wirkstofffrei ist (die Wirkstoffsättigung tritt während der Lagerung ein). In die Reservoirmatrix ist ein Wirkstoffdepot 14 eingebettet, das den Wirkstoff an Reservoirmatrix-Material abgibt und ihn dann durch die Fixierungseinrichtung 16 an die Haut 18 freisetzt. Das therapeutische System wird nach außen durch eine Rückschicht 10 abgeschlossen, die für den Wirkstoff und bevorzugt auch für Feuchtigkeit undurchlässig ist und gleichzeitig eine Stützfunktion für das System ausübt.

in Fig. 2 ist eine weitere Variante des erfindungsgemäßen Systems gezeigt, bei der auf einer Reservoirmatrixschicht 12 ein Wirkstoffdepot 14 liegt und durch eine Rückschicht 10 abgedeckt wird. Die Fixierungseinrichtung ist in dieser Zeichnung nicht dargestellt, da in diesem Fall die haftklebende Reservoirmatrix die Funktion der Fixiereinrichtung übernimmt. Diese Ausführungsform ist insofern vorteilhaft, als seine Herstellung denkbar einfach ist; es müssen lediglich definierte Mengen auf eine vorfabrizierte Matrixschicht aufgebracht und das ganze durch eine Rückschicht 10 abgeschlossen werden.

Typische Dickenabmessungen für erfindungsgemäße TTS sind: bei einer Gesamtdicke von ca. 123 µm bis 5550 µm, vorzugsweise 285 µm - 1550 µm; Dicke der Rückschicht 8 - 150 µm, vorzugsweise 15 - 100 µm; Dicke der mit dem Wirkstoffdepot in Verbindung stehenden Matrix: 100 - 5000 µm, vorzugsweise 200 - 1300 µm; Dicke der Schutzschicht: 15 - 400 µm, vorzugsweise 70 - 150 µm. Solche Schichtdicken werden annähernd erreicht, wenn man Flächenauftragsgewichte für die Matrix von 100 - 5000 g/m² wählt.

### Beispiel:

Es wird zunächst eine Haftklebermasse HS hergestellt durch Homogenisieren von
a) 933 g eines Handelsproduktes (^{®}Duro-Tak 387-2516 der Fa. Henkel, Düsseldorf, Deutschland - das ist eine 40 %ige Lösung eines selbstvernetzenden Acrylatpolymeren auf Basis von 2-Ethylhexylacrylat, Vinylacetat, Hydroxyethylacrylat, Glycidylmethacrylat und Titanchelatester in einem Lösungsmittelgemisch aus Essigsäureethylester, Ethanol, Heptan und Methanol) mit
b) 8 g eines Triglycerids fraktionierter Kokosfettsäuren (C₈-C₁₀; ^{®}Miglyol 812 der Fa. Evonik Witten, Deutschland).

Daneben werden 6210 g ^{®}Duro-Tak 387-2516, 553 g Essigsäureethylester und 311 g Ethanol mit 66 g des zuvor genannten Triglyerids sowie 626 g eines Acrylharzes aus Dimethylaminoethylmethacrylat und neutralen Methacrylsäureestern ^{®}Eudragit E 100 der Fa. Röhm-Pharma, Darmstadt, Deutschland) versetzt und homogenisiert (Klebermasse MS).

Daneben werden 72 g ^{®}Eudragit E 100 in 100 g Lavendelöl eingetragen und darin gelöst. Es resultiert die Wirkstoffzubereitung.

Die Haftklebermasse HS wird so auf eine abhäsiv ausgerüstete Schutzschicht (A) aufgetragen, dass nach Abdampfen der Lösemittel eine Haftkleberschicht mit einem Flächengewicht von 40 g/m2 gebildet wird.

Die Klebermasse MS wird so auf eine andere abhäsiv ausgerüstete Schutzschicht (B) aufgetragen, dass nach Abdampfen der Lösemittel ein Film mit einem Flächengewicht von 220 g/m² entsteht. Dieser Film wird auf die auf der Schutzschicht (A) aufgebrachte Haftkleberschicht aufkaschiert. Es resultiert die Unterbahn.

In einem weiteren Beschichtungsgang wird die Klebermasse MS so auf eine weitere abhäsiv ausgerüstete Schutzschicht (C) aufgetragen, dass nach Abdampfen der Lösemittel ein Film mit einem Flächengewicht von 110 g/m² entsteht, auf den die für den Wirkstoff undurchlässige Rückschicht aufkaschiert wird. Es wird dabei die Oberbahn gebildet.

Nach dem Abziehen der abhäsiv ausgerüsteten Schutzschicht (B) von der Unterbahn werden Ronden aus -Teefilterpapier (Flächengewicht 40 g/m²) mittig positioniert.

Anschließend wird die Wirkstoffzubereitung mittels eines eiförmigen Silikon-Schaumgummitampons mit einer Shore-Härte von 6 auf die Vliesstoffronden gedruckt. Die Menge der Wirkstoffzubereitung wird so bemessen, dass jedes TTS später 180 mg Lavendelöl enthält.

Die Oberbahn wird nach Abziehen der abhäsiv ausgerüsteten Schutzschicht (C) auf die Unterbahn (ausgerüstet mit Vliesstoffronden und dotiert mit Wirkstoffzubereitung) aufkaschiert, und es werden TTS ausgestanzt.

## Patentansprüche

1. Transdermales therapeutisches System (TTS), umfassend eine der Haut abgewandte, für den Wirkstoff undurchlässige Rückschicht,
a) ein Wirkstoffdepot, das einen Wirkstoff enthält, wobei der Wirkstoff Lavendelöl ist oder die reinen Wirkstoffe Linalool oder Linalylacetat sind,
b) eine Matrix, die mit dem Wirkstoffdepot in Verbindung steht und die Abgabe des Wirkstoffs steuert,
c) eine haftklebende Fixierungseinrichtung für das therapeutische System auf der Haut und
d) eine ablösbare Schutzschicht,
worin die Matrix und die haftklebende Fixiereinrichtung gleich oder verschieden sind und ein Material umfassen, das aus der Gruppe ausgewählt ist, die aus haftklebenden Polymeren auf Basis von Acrylsäure und/oder Metharylsäure sowie deren Estern, Polyacrylaten, Polyisobutylen, Polyvinylacetat, Ethylen-Vinylacetat-copolymer, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymeren und Heißschmelzklebern besteht, oder das auf Basis von haftklebenden Silikonpolymeren oder Polysiloxanen hergestellt ist,
wobei der Wirkstoffgehalt 30 bis 400 mg beträgt und
wobei das Wirkstoffdepot noch faserförmige Bestandteile umfassendes Stützmaterial enthält.

2. TTS gemäß Anspruch 1, worin das Wirkstoffdepot den reinen Wirkstoff enthält.

3. TTS gemäß Anspruch 1, worin das Wirkstoffdepot mindestens einen pharmazeutisch akzeptablen Hilfsstoff enthält.

4. TTS gemäß einem der Ansprüche 1 bis 3, welches als Wirkstoff 30 bis 400 mg, vorzugsweise 40 bis 300 mg, insbesondere 50 bis 150 mg Lavendelöl oder die reinen Wirkstoffe Linalool oder Linalylacetat enthält.

5. TTS gemäß einem der Ansprüche 1 bis 4, worin es sich bei dem Stützmaterial um Papier, textiles Material oder einen Vliesstoff handelt.

6. TTS gemäß einem der Ansprüche 1 bis 5, worin das Wirkstoffdepot eine Fläche von 10 bis 35 cm², vorzugsweise 12 bis 30 cm², insbesondere 15 bis 25 cm² aufweist.

7. TTS gemäß einem der Ansprüche 1 bis 6, worin die Rückschicht aus einem Material besteht, das aus der Gruppe ausgewählt ist, die Polyethylenterephthalat, weichgemachte Vinylacetat-Vinylchlorid-Copolymerisate, Nylon, Ethylen-Vinylacetat-Copolymerisate, weichgemachtes Polyvinylchlorid, Polyurethan, Polyvinylidenchlorid, Polypropylen, Polyethylen oder Polyamid umfasst.

8. TTS gemäß einem der Ansprüche 1 bis 7, worin die Matrix und die haftklebende Fixiereinrichtung gleich oder verschieden sind und ein Material umfassen, das aus der Gruppe ausgewählt ist, die aus kationischen Copolymeren auf Basis von Dimethylaminoethylmethacrylat und neutralen Methacrylsäureestern, und neutralen Copolymeren auf Basis von Butylmethacrylat und Methylmethacrylaten besteht.

9. Verfahren zur Herstellung eines TTS gemäß einem der Ansprüche 1 bis 8, **dadurch gekennzeichnet, dass** man
- ein Laminat aus einer wirkstoffundurchlässigen Trägerschicht, einer haftklebenden Fixierschicht und einer Matrixschicht herstellt,
- auf die Matrixseite dieses Laminats eine zur Aufnahme des Wirkstoffs vorgesehene Depotschicht aufbringt,
- mittels eines Druckverfahrens einzeln dosierte Portionen einer fliessfähigen, wirkstoffhaltigen Zubereitung auf diese Depotschicht aufbringt,
- darauf ggf. eine weitere Matrixschicht laminiert und
- das so erhaltene Laminat schließlich mit einer wirkstoffundurchlässigen Rückschicht versieht,
wobei die transdermalen therapeutischen Systeme durch Schneiden und/oder Stanzen vor oder nach dem Aufbringen der wirkstoffhaltigen Zubereitung aus dem bis dahin entstandenen Verbundlaminat vereinzelt werden können.

10. Verfahren gemäß Anspruch 9, **dadurch gekennzeichnet, dass** es sich bei dem Druckverfahren um ein Tampondruckverfahren handelt, oder um ein Verfahren, bei dem die wirkstoffhaltige Zubereitung durch eine mit mindestens einem Durchlass versehene Verteilerplatte einer Auftragsvorrichtung auf die zur Aufnahme des Wirkstoffs vorgesehene Depotschicht übertragen wird.

11. Verfahren gemäß einem der Ansprüche 9 bis 10, **dadurch gekennzeichnet, dass** die Viskosität der fließfähigen, wirkstoffhaltigen Zubereitung durch Zugabe von geeigneten Lösungsmittel und/oder Hilfsstoffen eingestellt wird.

12. Lavendelöl oder die reinen Wirkstoffe Linalool oder Linalylacetat zur Verwendung bei der Prophylaxe und/oder Behandlung von Unruhezuständen, Schlafstörungen, Ängsten und Nervosität, wobei ein TTS umfassend
a) eine der Haut abgewandte, für den Wirkstoff undurchlässige Rückschicht,
b) ein Wirkstoffdepot, das einen Wirkstoff enthält, wobei der Wirkstoff Lavendelöl ist oder die reinen Wirkstoffe Linalool oder Linalylacetat sind,
c) eine Matrix, die mit dem Wirkstoffdepot in Verbindung steht und die Abgabe des Wirkstoffs steuert und
d) eine haftklebende Fixierungseinrichtung für das therapeutische System auf der Haut,
worin die Matrix und die haftklebende Fixiereinrichtung gleich oder verschieden sind und ein Material umfassen, das aus der Gruppe ausgewählt ist, die aus haftklebenden Polymeren auf Basis von Acrylsäure und/oder Metharylsäure sowie deren Estern, Polyacrylaten, Polyisobutylen, Polyvinylacetat, Ethylen-Vinylacetat-copolymer, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymeren und Heißschmelzklebern besteht, oder das auf Basis von haftklebenden Silikonpolymeren oder Polysiloxanen hergestellt ist,
wobei der Wirkstoffgehalt 30 bis 400 mg beträgt und worin das Wirkstoffdepot noch faserförmige Bestandteile umfassendes Stützmaterial enthält,
auf die Haut aufgeklebt wird und der Wirkstoff in prophylaktisch bzw. therapeutisch wirksamer Menge transdermal abgegeben wird, vorzugsweise über einen Zeitraum von mindestens 2 und bis zu 48 Stunden.

## Claims

1. Transdermal therapeutic system (TTS) comprising a backing layer which is facing away from the skin and is impermeable for the active ingredient,
a) an active-ingredient depot containing an active ingredient, the active ingredient being lavender oil or being the pure active ingredients linalool or linalyl acetate,
b) a matrix which is in contact with the active-ingredient depot and controls the delivery of the active ingredient,
c) a pressure-sensitively adhering fixing means for the therapeutic system on the skin and
d) a detachable protective layer,
the matrix and the pressure-sensitively adhering fixing means being identical or different and comprising a material which is selected from the group consisting of pressure-sensitively adhering polymers based on acrylic acid and/or methacrylic acid and also their esters, polyacrylates, polyisobutylene, polyvinyl acetate, ethylene-vinyl acetate copolymer, natural and/or synthetic rubbers, for example acrylonitrile-butadiene rubber, butyl rubber or neoprene rubber, styrene-diene copolymers such as styrene-butadiene block copolymers and hotmelt adhesives, or which is produced on the basis of pressure-sensitively adhering silicone polymers or polysiloxanes,
the active-ingredient content being from 30 to 400 mg and
the active-ingredient depot additionally containing support material comprising fibrous constituents.

2. TTS according to Claim 1, wherein the active-ingredient depot contains the pure active ingredient.

3. TTS according to Claim 1, wherein the active-ingredient depot contains at least one pharmaceutically acceptable excipient.

4. TTS according to any of Claims 1 to 3, containing from 30 to 400 mg, preferably from 40 to 300 mg, more particularly from 50 to 150 mg, of lavender oil or the pure active ingredients linalool or linalyl acetate as active ingredient.

5. TTS according to any of Claims 1 to 4, wherein the support material is paper, textile material or a nonwoven.

6. TTS according to any of Claims 1 to 5, wherein the active-ingredient depot has an area of from 10 to 35 cm², preferably from 12 to 30 cm², more particularly from 15 to 25 cm².

7. TTS according to any of Claims 1 to 6, wherein the backing layer consists of a material selected from the group comprising polyethylene terephthalate, plasticized vinyl acetate-vinyl chloride copolymers, nylon, ethylene-vinyl acetate copolymers, plasticized polyvinyl chloride, polyurethane, polyvinylidene chloride, polypropylene, polyethylene or polyamide.

8. TTS according to any of Claims 1 to 7, wherein the matrix and the pressure-sensitively adhering fixing means are identical or different and comprise a material selected from the group consisting of cationic copolymers based on dimethylaminoethyl methacrylate and neutral methacrylic esters, and neutral copolymers based on butyl methacrylate and methyl methacrylates.

9. Method for producing a TTS according to any of Claims 1 to 8, **characterized in that**
- a laminate composed of an active-ingredient-impermeable support layer, a pressure-sensitively adhering fixing layer and a matrix layer is produced,
- a depot layer intended for the accommodation of the active ingredient is applied to the matrix side of said laminate,
- individually dispensed portions of a free-flowing, active-ingredient-containing preparation are applied to said depot layer by means of a printing process,
- optionally a further matrix layer is laminated thereon and
- the thus obtained laminate is lastly provided with an active-ingredient-impermeable backing layer,
it being possible to singularize the transdermal therapeutic systems from the hitherto formed composite laminate by cutting and/or punching before or after the application of the active-ingredient-containing preparation.

10. Method according to Claim 9, **characterized in that** the printing process is a pad printing process or a process in which the active-ingredient-containing preparation is transferred to the depot layer intended for the accommodation of the active ingredient by means of an application device distributor plate provided with at least one aperture.

11. Method according to any of Claims 9 to 10, **characterized in that** the viscosity of the free-flowing, active-ingredient-containing preparation is adjusted by addition of suitable solvents and/or excipients.

12. Lavender oil or the pure active ingredients linalool or linalyl acetate for use in the prophylaxis and/or treatment of restlessness, sleep disorders, anxieties and nervousness, wherein a TTS comprising
a) a backing layer which is facing away from the skin and is impermeable for the active ingredient,
b) an active-ingredient depot containing an active ingredient, the active ingredient being lavender oil or being the pure active ingredients linalool or linalyl acetate,
c) a matrix which is in contact with the active-ingredient depot and controls the delivery of the active ingredient and
d) a pressure-sensitively adhering fixing means for the therapeutic system on the skin,
the matrix and the pressure-sensitively adhering fixing means being identical or different and comprising a material which is selected from the group consisting of pressure-sensitively adhering polymers based on acrylic acid and/or methacrylic acid and also their esters, polyacrylates, polyisobutylene, polyvinyl acetate, ethylene-vinyl acetate copolymer, natural and/or synthetic rubbers, for example acrylonitrile-butadiene rubber, butyl rubber or neoprene rubber, styrene-diene copolymers such as styrene-butadiene block copolymers and hotmelt adhesives, or which is produced on the basis of pressure-sensitively adhering silicone polymers or polysiloxanes,
the active-ingredient content being from 30 to 400 mg and the active-ingredient depot additionally containing support material comprising fibrous constituents,
is adhered to the skin and the active ingredient is delivered transdermally in a prophylactically or therapeutically effective quantity, preferably over a period of at least 2 hours and up to 48 hours.

## Revendications

1. Système thérapeutique transdermique (STT), comprenant
une couche arrière imperméable à la substance active et tournée à l'opposé de la peau,
a) un dépôt de substance active qui contient une substance active, la substance active étant l'huile de lavande, ou les substances actives pures étant le linalol ou l'acétate de linalyle,
b) une matrice, qui est reliée au dépôt de substance active et qui contrôle la libération de la substance active,
c) un dispositif de fixation adhésif sensible à la pression pour le système thérapeutique sur la peau et
d) une couche de protection amovible,
dans lequel la matrice et le dispositif de fixation adhésif sensible à la pression sont identiques ou différents et comprennent un matériau qui est choisi dans le groupe constitué des polymères adhésifs sensibles à la pression à base d'acide acrylique et/ou d'acide méthacrylique et de leurs esters, des polyacrylates, du polyisobutylène, de l'acétate de polyvinyle, du copolymère éthylène-acétate de vinyle, des caoutchoucs naturels et/ou synthétiques, tels que le caoutchouc acrylonitrile-butadiène, le caoutchouc butyle ou le caoutchouc néoprène, des copolymères styrène-diène tels que les copolymères blocs styrène-butadiène, et des adhésifs thermofusibles, ou qui est fabriqué à base de polymères silicones adhésifs ou de polysiloxanes,
la teneur en substance active étant de 30 à 400 mg et
le dépôt de substance active contenant également un matériau de support comprenant des composants fibreux.

2. STT selon la revendication 1, dans lequel le dépôt de substance active contient la substance active pure.

3. STT selon la revendication 1, dans lequel le dépôt de substance active contient au moins un excipient pharmaceutiquement acceptable.

4. STT selon l'une des revendications 1 à 3, qui contient de 30 à 400 mg, de préférence de 40 à 300 mg, notamment de 50 à 150 mg d'huile de lavande ou les principes actifs purs linalol ou acétate de linalyle à titre de substance active.

5. STT selon l'une des revendications 1 à 4, dans lequel le matériau de support est du papier, un matériau textile ou un tissu non tissé.

6. STT selon l'une des revendications 1 à 5, dans lequel le dépôt de substance active a une surface de 10 à 35 cm², de préférence de 12 à 30 cm², notamment de 15 à 25 cm².

7. STT selon l'une des revendications 1 à 6, dans lequel la couche arrière est constituée d'un matériau choisi dans le groupe constitué par le polyéthylène téréphtalate, les copolymères plastifiés d'acétate de vinyle-chlorure de vinyle, le nylon, les copolymères éthylène-acétate de vinyle, le polychlorure de vinyle plastifié, le polyuréthane, le chlorure de polyvinylidène, le polypropylène, le polyéthylène ou le polyamide.

8. STT selon l'une des revendications 1 à 7, dans lequel la matrice et le dispositif de fixation adhésif sensible à la pression sont identiques ou différents et comprennent un matériau qui est choisi dans le groupe constitué par les copolymères cationiques à base de méthacrylate de diméthylami-noéthyle et d'esters neutres d'acide méthacrylique, et les copolymères neutres à base de méthacrylate de butyle et de méthacrylates de méthyle.

9. Procédé de fabrication d'un STT selon l'une des revendications 1 à 8, **caractérisé en ce que**
- l'on produit un produit laminé d'une couche de support imperméable à la substance active, une couche de fixation adhésive sensible à la pression et une couche de matrice,
- l'on applique une couche de dépôt destinée à absorber la substance active sur la face matrice de ce produit laminé,
- l'on applique des portions dosées individuellement d'une préparation fluide contenant la substance active sur cette couche de dépôt au moyen d'un processus d'impression,
- l'on lamine éventuellement un autre couche de matrice sur celle-ci et
- l'on munit enfin le produit laminé ainsi obtenu d'une couche
support imperméable à la substance active,
dans lequel les systèmes thérapeutiques transdermiques peuvent être séparés du produit composite laminé précédemment créé par découpe et/ou poinçonnage avant ou après l'application de la préparation contenant la substance active.

10. Procédé selon la revendication 9, **caractérisé en ce que** le procédé d'impression est un procédé de tampographie, ou un procédé dans lequel la préparation contenant la substance active est transférée à travers une plaque de distribution d'un dispositif d'application muni d'au moins un passage sur la couche de dépôt prévue pour recevoir la substance active.

11. Procédé selon l'une des revendications 9 à 10, **caractérisé en ce que** la viscosité de la préparation fluide contenant la substance active est ajustée en ajoutant des solvants et/ou des excipients appropriés.

12. Huile de lavande ou les principes actifs purs linalol ou acétate de linalyle pour une utilisation dans la prophylaxie et/ou le traitement de l'agitation, des troubles du sommeil, de l'anxiété et de la nervosité, dans laquelle un STT comprenant
a) une couche arrière imperméable à la substance active et tournée à l'opposé de la peau,
b) un dépôt de substance active qui contient une substance active, la substance active étant l'huile de lavande, ou les substances actives pures étant le linalol ou l'acétate de linalyle,
c) une matrice, qui est reliée au dépôt de substance active et qui contrôle la libération de la substance active et
d) un dispositif de fixation adhésif sensible à la pression pour le système thérapeutique sur la peau,
la matrice et le dispositif de fixation adhésif sensible à la pression étant identiques ou différents et comprenant un matériau qui est choisi dans le groupe constitué des polymères adhésifs sensibles à la pression à base d'acide acrylique et/ou d'acide méthacrylique et de leurs esters, des polyacrylates, du polyisobutylène, de l'acétate de polyvinyle, du copolymère éthylène-acétate de vinyle, des caoutchoucs naturels et/ou synthétiques, tels que le caoutchouc acrylonitrile-butadiène, le caoutchouc butyle ou le caoutchouc néoprène, des copolymères styrène-diène tels que les copolymères blocs styrène-butadiène, et des adhésifs thermofusibles, ou qui est fabriqué à base de polymères silicones adhésifs ou de polysiloxanes,
la teneur en substance active étant de 30 à 400 mg et le dépôt de substance active contenant également un matériau de support comprenant des composants fibreux,
est collé sur la peau et la substance active est administré par voie transdermique dans une dose prophylactiquement ou thérapeutiquement efficace, de préférence pendant une période d'au moins 2 heures et jusqu'à 48 heures.
